# EUROPÄISCHE PATENTSCHRIFT

(11) **EP 1 109 522 B1**
(45) Veröffentlichungstag und Bekanntmachung des Hinweises auf die Patenterteilung: **29.04.2009**
(21) Anmeldenummer: 00938435.5
(22) Anmeldetag: 30.06.2000
(51) Int. Cl.: A61F 13/66

(54) **SCHUTZHOSE UND VERFAHREN ZUR HERSTELLUNG DERSELBEN**
PROTECTIVE UNDERPANTS AND METHOD FOR PRODUCING THE SAME
CULOTTE DE PROTECTION ET PROCEDE DE FABRICATION DE LADITE CULOTTE

(30) Priorität: 01.07.1999 CH 121699
(43) Veröffentlichungstag der Anmeldung: 27.06.2001
(73) Patentinhaber: I.M.E.C. medizinische Textilien GmbH, 9010 St. Gallen (CH)
(72) Erfinder: HALID, Michael, CH-9010 St. Gallen (CH)
(74) Vertreter: Mötteli-Mantelli, Novella
(86) Internationale Anmeldenummer: PCT/CH2000/000354
(87) Internationale Veröffentlichungsnummer: WO 2001/001911

(56) Entgegenhaltungen:
- WO-A-96/36248
- WO-A-98/43503
- CH-A- 442 611
- DE-C- 821 102
- US-A- 2 977 957
- US-A- 4 690 681
- US-A- 4 695 279
- US-A- 5 546 607
- US-A- 5 669 902
- US-A- 5 745 922

## Beschreibung

Die vorliegende Erfindung betrifft eine Schutzhose sowie ein Verfahren zur Herstellung derselben.

Eine Schutzhose ist aus WO 98/43503 bekannt. Diese Schutzhose ist eine Unterhose und sie weist eine sogenannte Barriere für eine Flüssigkeit in jenem Bereich derselben auf, welcher dem Genitalbereich des Benützers der Hose zugeordnet ist. Diese Barriere ist aus aufeinander liegenden Materialschichten zusammengesetzt, von welchen eine die Flüssigkeit aufnehmen kann. Die Barriere ist für eine Flüssigkeit praktisch undurchlässig, sie nimmt einen Bereich der Vorderseite der Unterhose ein und sie ist in diesem Bereich der Hose eingenäht, sodass diese Barriere einen Abschnitt der Vorderwand der Hose darstellt. Die einzelnen Schichten dieser Barriere sind verhältnismässig dünn. Wenn die Menge der aus dem Körper des Trägers dieser Hose austretenden Flüssigkeit eine bestimmte Grösse überschreitet, dann kann die dünne Barriere diese Menge der Flüssigkeit nicht aufnehmen und die nicht aufgenommene Menge der Körperflüssigkeit tritt aus der Hose heraus. Dies um so mehr, als die Flüssigkeit auch durch die Nähte dieser Unterhose durchsickern kann, mit deren Hilfe die Barriere an den übrigen Teil der Schutzhose angeschlossen ist.

Die Aufgabe der vorliegenden Erfindung ist, die genannten Nachteile sowie noch weitere Nachteile des Standes der Technik zu beseitigen.

Gegenstand der Erfindung ist eine Schutzhose gemäß Patentanspruch 1 und ein Verfahren zur Herstellung einer Schutzhose gemäß Patentanspruch 13.

Nachstehend werden Ausführungsformen der vorliegenden Erfindung anhand der beiliegenden Zeichnungen näher erläutert. Es zeigt:
Fig. 1 in einer Frontansicht eine erste Ausführung der vorliegenden Schutzhose, deren Grundkörper aus einem Aussenteil und aus einem Innenteil zusammengesetzt ist,
Fig. 2 bis 4 in einem horizontal verlaufenden Schnitt Ausschnitte aus drei Bereichen der Schutzhose gemäss Fig. 1,
Fig. 5 in einer Frontansicht den Innenteil des Grundkörpers der Schutzhose gemäss Fig. 1,
Fig. 6 in einer rückwärtigen Ansicht den Innenteil aus Fig. 5,
Fig. 7 in einer Draufsicht einen Ausschnitt aus der Innenseite im Schrittbereich des Hoseninnenteiles gemäss Fig. 5 und 6,
Fig. 8 in einem vertikalen Längsschnitt die Schutzhose gemäss Fig. 1,
Fig. 9 bis 16 in einer Frontansicht weitere Ausführungsformen der Schutzhose,
Fig. 17 in einem horizontal verlaufenden Schnitt einen Ausschnitt aus dem Schrittbereichen einer Mittelpartie der Schutzhose gemäss Fig. 13 und
Fig. 18 in einem vertikal verlaufenden Schnitt eine Verbindungsstelle innerhalb einer zweiteiligen Mittelpartie der Schutzhose.

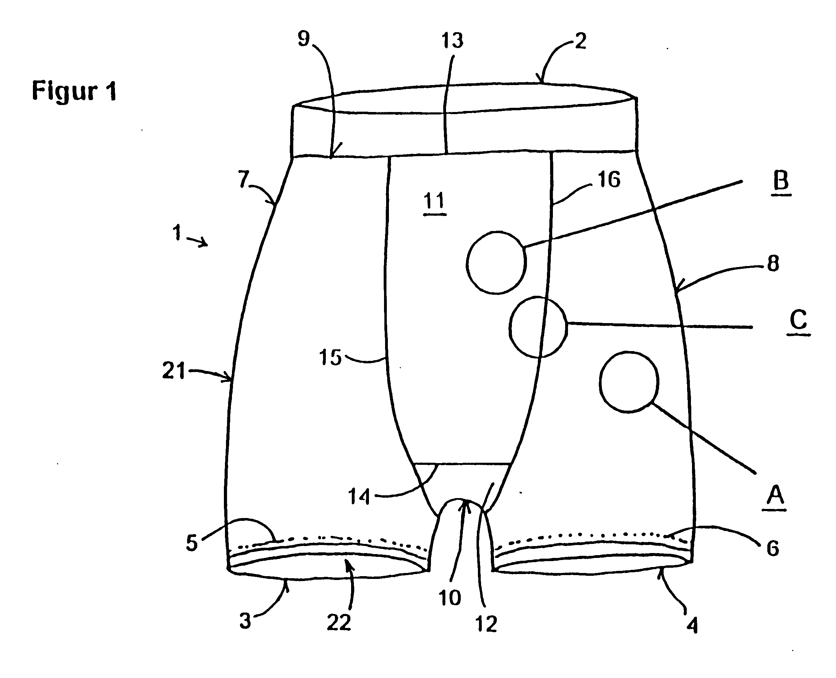

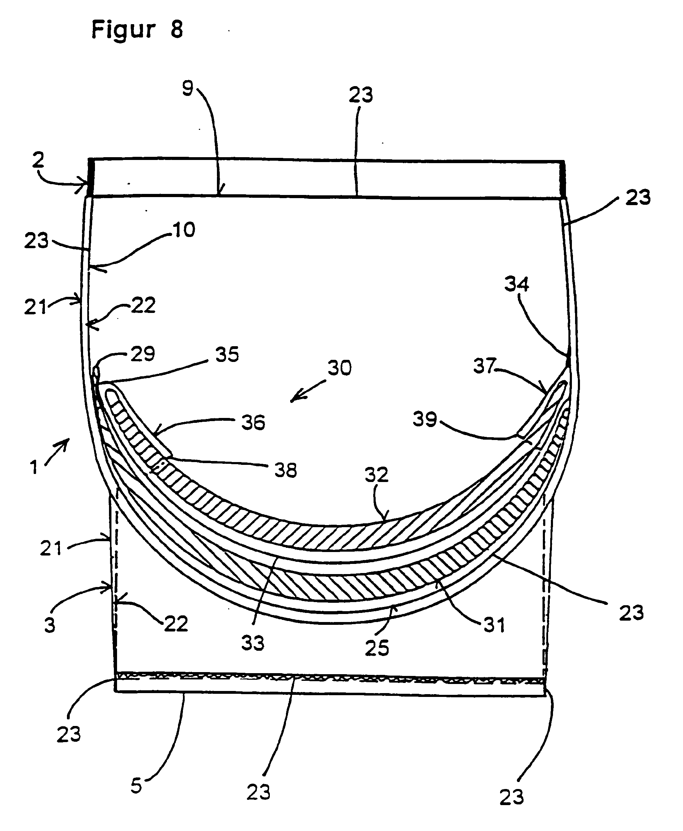

Die in Fig. 1 und 8 dargestellte Schutzhose weist einen Grundkörper 1 auf, dessen Form der Form des Grundkörpers einer üblichen Unterhose entspricht. An die oben liegende Oeffnung 9 des Hosengrundkörpers 1 ist ein an sich bekannter elastischer Bund 2 angeschlossen, beispielsweise durch Annähen. Dieser Bund 2 ist im dargestellten Fall als ein flaches Band ausgeführt, welches über eine seiner Längskanten an den Hosengrundkörper 1 angeschlossen ist. Bei der in Fig. 1 dargestellten Hose weist der Hosengrundkörper 1 Beine 3 und 4 auf. Im freien Randbereich dieser Beine 3 und 4 sind an sich bekannte Beinnähte 5 und 6 ausgeführt.

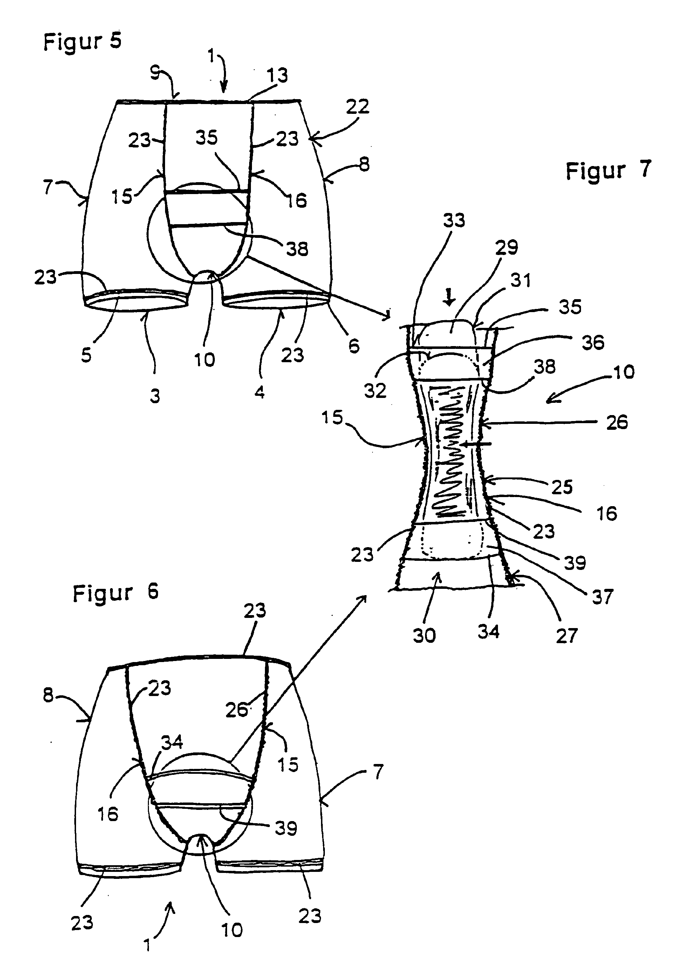

Der Hosengrundkörper 1 besteht aus einem Aussenteil 21 und aus einem Innenteil 22, welche im wesentlichen gleich ausgebildet sind. Der Innenteil 22 ist im Inneren des Aussenteiles 21 angeordnet. In Fig. 1 ist die vorliegende Schutzhose im fertiggestellten Zustand dargestellt und deswegen ist der Innenteil 22 des Hosengrundkörpers 1 aus Fig. 1 nicht ersichtlich. Dieser Innenteil 22 ist Fig. 8 zusammen mit dem Aussenteil 21 und in Fig. 5 bis 7 allein dargestellt.

Die vorliegende Schutzhose wird in der Weise hergestellt, dass der Aussenteil 21 und der Innenteil 22 des Grundkörpers 1 der Schutzhose getrennt hergestellt werden. Dann wird der Innenteil 22 in den Aussenteil 21 gelegt und diese Teile 21 und 22 werden in den Bereichen des Bundes 2 und der Hosenbeinöffnungen 3 und 4 zusammen verbunden. Dabei kann der Bund 2 an den Grundkörper 1 zugleich angeschlossen werden.

Der jeweilige Teil 21 bzw. 22 des Grundkörpers 1 der Hose umfasst zwei Seitenteile 7 und 8 sowie einen dazwischen angeordneten Mittelteil 10. Die Seitenteile 7 und 8 des Grundkörpers 1 sind zum Aufliegen an den Seiten des menschlichen Rumpfes im Bereich des Beckens desselben bestimmt. Der Umriss dieser Seitenteile 7 und 8 ist auch dementsprechend geformt. Die Aussenkontur von Zuschnitten (nicht dargestellt), aus welchen die Seitenteile 7 und 8 entstehen, weist einen ersten und beinahe geradling verlaufenden Abschnitt auf, welcher sich im Taillenbereich 2 der Hose befindet. Dieser Abschnitt des Umrisses des Zusschnittes liegt auf der die obere Oeffnung 9 des Hosengrundkörpers 1 begrenzenden Linie.

Von den Enden des ersten Konturabschnittes bzw. der Taillenabschnittes der Seitenteile 7 und 8 laufen je zwei weitere Abschnitte 15 und 16 der Kontur des jeweiligen Seitenteiles 7 bzw. 8 herab, über welche die Seitenteile 7 und 8 mit dem Mittelteil 10 verbunden sind. Die Längskanten des Mittelteiles 10, über welche dieser an die Seitenteile 7 und 8 angeschlossen ist, haben eine Form, deren Verlauf dem Verlauf der genannten Konturabschnitte 15 und 16 der Seitenteile 7 und 8 entspricht. Wenn die Hose Beine 3 und 4 aufweist, wie dies bei der Hose gemäss Fig. 1 bis 8 der Fall ist, dann verlaufen die zweiten bzw. weiteren Abschnitte 15 und 16 der Kontur der Seitenteile 7 und 8, dass sie jenes Material umgeben, aus welchem unter anderem auch die Hosenbeine bestehen. Die Konturen der Seitenteile 7 und 8 sind spiegelbildlich ausgeführt, wobei diese Konturen sonst zumindest im wesentlichen gleich sein können.

Der Mittelteil 10 verläuft durch den Schrittbereich der Hose und er ist aus zwei länglichen und hintereinander geschalteten Abschnitten 11 und 12 zusammengesetzt. Diese Abschnitte 11 und 12 weisen unterschiedliche Längen auf. Der an der Vorderseite der Hose liegende Mittelteilabschnitt 11 ist kürzer als der überwiegend im hinteren Bereich der Hose liegende Mittelteilabschnitt 12. Die Querkanten 13, welche sich an den freien Enden der Mittelteilabschnitte 11 und 12 befinden, liegen ebenfalls auf jener Linie, welche die obere Oeffnung 9 des Hosengrundkörpers 1 begrenzt. Eine dieser Endkanten 13 liegt im vorderen Bereich der Hose und die andere Endkante 13 befindet im hinteren Bereich der Hose. Die jeweilige Endkante 13 des Mittelteiles 10 liegt zwischen den Taillenabschnitten der Kontur des Zuschnittes der Seitenteile 7 und 8, sodass die Endkanten 13 des Mittelteiles 10 die Lücken zwischen den Taillenabschnitten der Kontur der Seitenteile 7 und 8 schliessen und die obere Oeffnung 9 im Hosengrundkörper 1 mitbegrenzen. Der so zusammengesetzte obere Rand 9 des Hosengrundkörpers 1 ist an den unteren Rand des Bundes 2 angeschlossen.

Die innere Kante 14 des vorderen Mittelteilabschnittes 11 liegt im vorderen Bereich der Hose, weil der Vorderabschnitt 11 des Mittelteiles 10, wie dies vorstehend dargelegt ist, kürzer ist als der Hinterabschnitt 12 des Mittelteiles 10. Die Innenkante ist mit der inneren Querkante des hinteren und längeren Mittelteilabschnittes 12 verbunden, sodass diese Innenkante in Fig. 1 nicht gesondert dargestellt sein kann. Entlang den Längsrändern 15 und 16 des Mittelteiles 10 erstrecken sich Verbindungsstellen zwischen dem Mittelteil 10 und einem der Seitenteile 7 bzw. 8 der Hose. Diese Verbindungsstellen 15 und 16 können beispielsweise als Nähte ausgeführt sein. Die Ränder 13, 15 und 16 des Mittelteiles 10 sind sowohl an den Bund 2 als auch an die zugewandten bzw. diesem Mittelteil 10 zugeordneten Ränder der Seitenteile 7 und 8 angeschlossen.

Für die genanten Anschlüsse bzw. für die Verbindungen unter den einzelnen Abschnitten 2, 7, 8, 10, 11 und 12 des Grundkörpers 1 der Hose untereinander kommen auch noch andere Techniken als Nähen in Frage, nämlich beispielsweise Kleben, Schweissen usw.

Fig. 5 zeigt in einer Frontansicht den Innenteil 22 des Grundkörpers 1 der vorliegenden Schutzhose. Fig. 6 zeigt in einer rückwärtigen Ansicht den genannten Innenteil 22. Aus Fig. 7 ist ein Ausschnitt aus der Innenseite des Schrittbereiches des Innenteiles 22 gemäss Fig. 5 und 6 in einer Draufsicht ersichtlich. Fig. 8 zeigt in einem vertikalen Längsschnitt die Schutzhose gemäss Fig. 1.

Wie erwähnt, können der Aussenteil 21 und der Innenteil 22 des Grundkörpers 1 der vorliegenden Hose gleich ausgebildet, wobei Fig. 8 zeigt, wie die Innenhose 22 in der Aussenhose 21 angeordnet ist. Der Innenteil 22 weist ebenfalls die Seitenteile 7 und 8 sowie den Mittelteil 10 auf. Im dargestellten Fall ist der Mittelteil 10 der Innenhose 22 einstückig, sodass dieser Mittelteil 10 keine Quernaht 14 aufweist. Auch die Innenhose 22 hat die Längsverbindungen 15 und 16, welche sich entlang den einander zugeordneten Stellen an den Kanten der Seitenteile 7 bzw. 8 und an den Kanten des Mittelteiles 10 erstrecken.

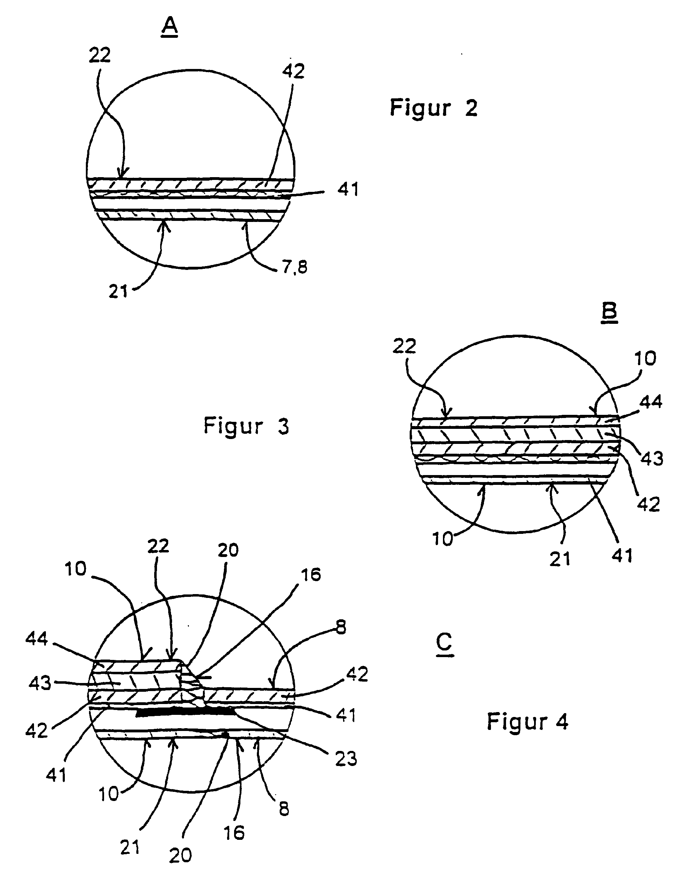

Die Teile 21 und 22 der vorliegenden Schutzhose sind nur im Bereich des Bundes 2 und der unteren Oeffnungen 5 und 6 der Hose miteinander verbunden. Dazwischen liegen die Materialien der Hosenteile 21 und 22 lose auf- bzw. aneinander, wie dies beispielsweise aus Fig. 2 bis 4 ersichtlich ist.

Es sind Abdichtbänder 23 (Fig. 5 bis 8) vorgesehen, welche unter anderem die Nahtstellen überdecken, an welchen zwei Bestandteile der Innenhose 22 aneinander treffen. Die Abdichtstreifen 23 überdecken bei der Innenhose 22 unter anderem auch die Nahtstellen 15 und 16 zwischen dem Mittelteil 10 und den Seitenteilen 7 und 8 sowie die Nahtstelle 14 innerhalb des Innenteiles 10. Die Abdichtstreifen 23 sind an der Aussenseite des Innenteiles 22 der voliegenden Schutzhose angebracht. Solche Abdichtstreifen 23 befinden sich auch im Bereich des Bundes 2 sowie im Bereich der Hosenbeine 3 und 4, und zwar zwischen der Aussenhose 21 und der Innenhose 22, wo sie jene Stellen der Schutzhose abdichten, an welchen die Ränder der Aussenhose 21 und des Innenhose 22 aufeinander aufliegen.

Die Abdichtbänder 23 können als Klebebänder oder als Bänder aus einem Material sein, welches unter der Einwirkung von Wärme weich bzw. halbflüssig wird. Das erweichte Material dringt in die darunter liegenden Vertiefungen ein, füllt diese aus und nach Abkühlen stellt das Material solcher Streifen 23 sicher, dass die Nahtstellen zwischen zwei benachbarten Bestandteilen eines der Teile 21 bzw. 22 der Schutzhose sowie die Spalte zwischen den Hosenteilen 21 und 22 flüssigkeitsdicht sind.

Die Umfangskontur des Mittelteiles 10 (Fig. 7) der Aussenhose 21 und der Innenhose 22 hat im wesentlichen die Form des Umrisses einer Sanduhr, wobei in Fig. 7 nur der Mittelabschnitt des Mittelteiles 10 dargestellt ist. Diese Aussenkontur des Mittelteiles 10 hat eine engste Stelle 25, welche sich unmittelbar im Schrittbereich der Schutzhose befindet. An die jeweilige Endpartie dieser engsten Stelle 25 schliessen sich erweiterte Abschnitte 26 und 27 der genannten Kontur des Mittelteiles 10 an. Am Ende des jeweiligen Breitabschnittes 26 bzw. 27 des Mittelteiles 10 befindet sich jeweils eine der bereits erwähnten Quer- bzw. Endkanten des Mittelteiles 10, welche auf der die obere Oeffnung 9 des Hosengrundkörpers 1 liegenden Linie liegt. Zwischen diesen Endkanten des Mittelteiles 10 erstrecken sich die Längskanten 15 und 16, deren Verlauf bereits beschrieben worden ist.

Die Innenseite, d.h. die dem Körper zugewandte Seite des Mittelteiles 10 der Innenhose 22 weist Mittel 30 zur Halterung von übereinander liegenden länglichen Einlagen 31 und 32 auf, welche einer an sich bekannten Art sein können. Der erste Teil der Haltemittel 30 ist als ein längliches Fach ausgeführt (Fig. 7 und 8), wobei die Längsrichtung dieses Faches mit der Längsrichtung des Mittelteiles 10 zusammenfällt. Dieses Fach ist durch eine Deckwand 33 aus einem textilen Stoff gebildet, welches auf der Ober- bzw. Innenseite der Mittelpartie 25 des Mittelteiles 10 aufliegt. Die Endpartien dieser Deckwand 33 liegen im Bereich der breiten Partien 26 und 27 (Fig. 7) des Mittelteiles 10 und sie überdecken dabei die sich an die engste Stelle 25 anschliessenden Abschnitte der breiten Partien 26 und 27 des Mittelteiles 10.

Der Verlauf der Längsränder dieser Deckwand 33 entspricht dem Verlauf der Kanten 15 und 16 des Mittelteiles 10, wobei diese Ränder der Deckwand 33 mit dem Material des Mittelteiles 10 der Innenhose 22 verbunden sind, beispielsweise durch Nähen. Die im hinteren Bereich der Schutzhose liegende und quer zur Längsrichtung des Mittelteiles 10 verlaufende Endkante 34 der Deckwand 33 ist mit dem Material des Mittelteiles 10 verbunden, und zwar beispielsweise ebenfalls durch Nähen. Die im vorderen Bereich der Hose liegende Vorderkante 35 der Deckwand 33 ist an den Mittelteil 10 nicht angeschlossen, sodass hier eine Oeffnung vorhanden ist, durch welche die erste Einlage 31 in dieses Fach 33 eingeführt werden kann. Das hintere Ende dieser ersten Einlage 31 liegt dann im Bereich der Hinterkante 34 dieses Faches 33. Die Länge der Deckwand 33 und somit auch dieses Faches ist im dargestellten Fall kleiner alls die Länge der Einlage 31, sodass ein Abschnitt 29 der ersten Einlage 31 vorne aus dem Fach 33 hervorsteht. Die Länge der Deckwand 33 kann jedoch auch gleich gross sein wie die Länge der Einlage 31.

Für die Halterung der zweiten und oberhalb der ersten Einlage 31 liegenden Einlage 32 sind Taschen 36 und 37 vorgesehen, welche für die Aufname der Endpartien der zweiten Einlage 32 bestimmt und ausgebildet sind. Die jeweilige Tasche 36 bzw. 37 ist durch einen im wesentlichen viereckförmigen Abschnitt aus einem textilen Stoff gebildet, welcher auf der dem Körper zugewadten Seite der Deckwand 33 des Faches befestigt ist.

Der erste Stoffabschnitt 36 ist auf dem vorderen Abschnitt der Deckwand 33 angebracht, wo sich die Einlegeöffnung 35 für die erste Einlage 31 befindet. Dabei ist die vorne liegende Kante dieses ersten Stoffabschnittes 36 mit der frei stehenden Vorderkante 35 der Deckwand 33 praktisch bündig. Die Vorderkante und die Seitenkanten dieses ersten Stoffabschnittes 36 sind mit dem Stoff der Deckwand 33 verbunden, beispielsweise durch Nähen. Da jene Kante 29 dieses Stoffabschnittes 36 an die Deckwand 33 nicht angeschlossen ist, welche dem anderen Ende der Deckwand 33 näher liegt, weist diese Tasche 36 hier eine Oeffnung auf, durch welche eine der Endpartien der zweiten Einlage 32 in diese Tasche 36 eingeführt werden kann.

Der zweite Stoffabschnitt 37 ist auf dem hinten liegenden Abschnitt der Deckwand 33 angebracht, wo sich die an das Material der Innenhose 22 angeschlossene hintere Querkante 34 des Faches 33 befindet. Die hinten liegende Kante dieses Stoffabschnittes 37 ist zusammen mit der hier liegenden Hinterkante 34 der Deckwand 33 an das Material der Innenhose 22 angeschlossen. Da die Vorderkante 39 dieses Stoffabschnittes 37 weder mit der Deckwand 33 noch mit dem Material der Innenhose 22 verbunden ist, ist eine Oeffnung dieser Tasche 37 im Bereich der genannten Vorderkante 39 vorhanden und durch diese Oeffnung kann die zweite Endpartie der zweiten Einlage 32 in diese Tasche 37 eingeführt werden. Die zweiten Einlage 32 ist kürzer als der Abstand zwischen den Kanten 34 und 35 der Deckwand 33, sodass diese Einlage 32 in die so ausgeführte Haltevorrichtung ohne Probleme passt. Die zweite Einlage 32 ist im dargestellten Fall kürzer als die erste Einlage 31. Diese Einlagen 31 und 32 können jedoch auch gleich lang und auch sonst identisch sein.

Fig. 2 bis 4 zeigen in einem horizontal verlaufenden Schnitt Ausschnitte aus drei Bereichen der Schutzhose gemäss Fig. 1. Fig. 2 zeigt einen Ausschnitt aus einem der Seitenbereiche 7 bzw. 8 der Schutzhose. Im unteren Abschnitt des jeweiligen Seitenbereiches der Schutzhose kann sich eines der Hosenbeine 3 bzw. 4 befinden.

Der Ausschnitt gemäss Fig. 2 umfasst einen Ausschnitt aus der Aussenhose 21 und einen Ausschnitt aus der Innenhose 22, welche praktisch parallel zueinander verlaufen. Damit es deutlicher ist, dass es sich um zwei ineinander gelegte Teilhosen 21 und 22 handelt, sind die Ausschnitte aus diesen Teilhosen 21 und 22 in einem Abstand voneinander dargestellt. Die Seitenteile 7 und 8 der Aussenhose 21 sind durch einen Oberstoff gebildet, welcher bei der Herstellung von Unterhosen normalerweise verwendet wird. Folglich sieht die vorliegenden Schutzhose auf den ersten Blick zunächst als eine gewöhnliche Unterhose aus. Dieser Stoff kann mit Vorteil ein Baumwollstoff sein.

Das Material der Aussenteile 7 und 8 der Innenhose 22 umfasst dagegen einen Sicherheitsstoff, welcher im vorliegenden Fall zwei Schichten aus unterschiedlichen Materialien aufweist. An der Aussenseite der Innenhose 22 befindet sich eine Schicht 41 aus einem flüssigkeitsundurchlässigen Stoff. Diese Schicht 41 kann beispielsweise als eine Membrane aus Polyuretan ausgeführt sein, weil eine solche Membrane zwar flüssigkeitsundurchlässig jedoch atmungsaktiv ist. Diese Aussenschicht 41 befindet sich an der der Aussenhose 21 zugewandten Seite der Innenhose 22. An der Innenseite des Aussenteiles 7 bzw. 9 der Innenhose 22 befindet sich ein Innenstoff 42, welcher saugfähig und hautfreundlich ist. Dieser Stoff 42 kann aus Faden sein, welche ein Gemisch aus Baumwoll- und Polyesterfasern oder aus Baumwoll- und Polyamidfasern enthalten. Dabei kann es sich um an sich bekannte Gemische aus den genannten Fasern handeln.

Fig. 3 zeigt einen Ausschnitt aus dem Mittelbereich der Schutzhose, wo sich der Mittelteil 10 sowohl bei der Aussenhose 21 als auch bei der Innenhose 22 befindet. Der Mittelteil 10 der Aussenhose 21 ist aus demselben Stoff wie die Seitenteile 7 und 8 der Aussenhose 21, wie dies im Zusammenhang mit Fig. 2 beschrieben ist. Da der Hauptteil der aus dem Körper des Benützers der Schutzhose austretenden Flüssigkeit im Mittelteile 10 der Innenhose 22 gespeichert werden soll, weist der Sicherheitsstoff dieses Mittelteiles 10 eine mehrschichtige Struktur auf. An der Aussenseite der Innenhose 22 liegt der bereits genannte flüssigkeitsdichte Stoff 41. An der Innenseite dieser Aussenschicht 41 ist der vorstehend ebenfalls bereits genannte Innenstoff 42 angeordnet, welcher Baumwolle und Polyester enthalten kann.

An der Innenseite des Innenstoffes 42 befindet sich ein Stoff 43, welcher eine möglichst grosse Saugfähigkeit bzw. Kapazität zur Speicherung der austretenden Flüssigkeit aufweist. Diese Stoffschicht 43 kann beispielsweise aus einem Doppelfrottee sein. Ausserdem kann diese Stoffschicht entweder geruchshemmend ausgerüstet sein oder/und sie kann mit geruchshemmenden Garnen gewoben, gestrickt oder gewirkt sein.

Die dem Körper zugewandte Oberfläche der Saugstoffschicht 43 ist mit einer weiteren Materialschicht 44 versehen. Diese Materialschicht 44 ist aus einem Stoff, welcher auch als Transportstoff bezeichnet werden kann. Dieser Stoff 44 kann ebenfalls aus geruchshemmenden Garnen hergestellt sein, wie z.B. aus Polyester oder aus Mikrofasern. Dieser Stoff lässt Flüssigkeiten nur von seiner freien Oberfäche her durch das Innere dieses Sicherheitsstoffes 44 fliessen, wodurch eine Rücknässung der Haut des Benützers der Schutzhose durch eine Rückfluss der Flüssigkeit aus der Schicht 43 verhindert wird. Die Haut des Benützers bleibt somit im wesentlichen trocken. Die dem Körper des Benützers zugewandte Oberfläche dieses Transportstoffes 44 ist hautfreundlich. Der Stoff 44 selbst ist derart, dass er am Körper des Trägers der Schutzhose nicht haften kann, nachdem die Flüssigkeit aus einer Körperöffnung ausgetreten war und danach austrocknete.

In Fig. 4 ist ein Ausschnitt aus einer der Uebergangspartien zwischen einem der Seitenteile 7 bzw. 8 und dem Mittelteil 10 der Aussenhose 21 und der Innenhose 22 dargestellt. Der dargestellte Seitenteil 8 und der Mittelteil 10 der Aussenhose 21 sind aus dem genannten konventionellen Stoff für eine Unterhose hergestellt, wie dies vorstehend beschrieben ist. Der dargestellte Seitenteil 8 der Innenhose 22 weist nur die zwei vorstehend bereits beschriebenen Schichten 41 und 42 auf. Der Mittelteil 10 der Innenhose 21 weist alle vier im vorstehenden beschriebenen Schichten 41 bis 44 auf. Die Verbindung der Teile 8 und 10 der Aussenhose 21 im Bereich der Verbindungsstelle 16 an der Aussenhose 21 erfolgt mit Hilfe einer Naht 20. Der Mittelteil 10 und der Seitenteil 8 der Innenhose 22 sind im Bereich der Verindungsstelle 16 ebenfalls mit Hilfe einer Naht 20 miteinander verbunden. An der Aussenseite der Naht 20 bzw. der Verbindungsstelle 16 der Innenhose 22 ist der bereits beschriebene Abdichtstreifen 23 angebracht, welcher breiter ist als die Naht 20.

Es versteht, dass diese Darlegungen auch für die übrigen Partien der Schutzhose in einer analogen Weise zutreffen. Die Abdichtstreifen 23 befinden sich auch im Bereich des Bundes 2 und der Hosenbeine 3 und 4, wie dies bereits erwähnt worden ist. Diese Streifen 23 können beispielsweise aus Polyuretan sein, welcher unter der Einwirkung von Hitze weich bis zähflüssig wird.

Die vorliegende Schutzhose kann als ein Unterwäschestück, als eine Sporthose, eine medizinische Hose usw. Verwendung finden. Sie ist flüssigkeitsundurchlässig, saugfähig, verschweisst und trotzdem atmungsaktiv. Ausserdem ist die Schutzhose so ausgeführt, dass sie ihre nützlichen Eigenschaften auch nach mehrmaligem Waschen beibehält. Diese Hose kann von leicht-, mittel- und schwerinkontinenten jedoch nicht betlägrigen Menschen getragen werden, wobei die Verwendung der vorstehend beschriebenen Einlagen 31 und 32 vor allem bei schwerer Inkontinenz angezeigt ist.

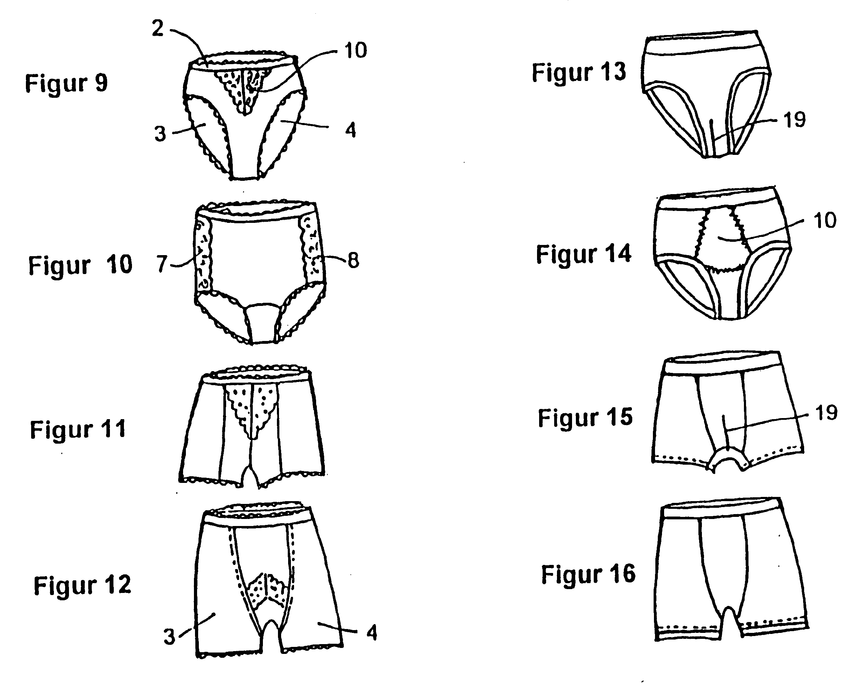

Solche Schutzhosen können unterschiedliche Schnitte aufweisen. Durch diese Schnitte kann unterschieden werden unter den Schutzhosen für Frauen, Männer und Kinder, wobei hier ebenfalls unterschieden werden kann zwischen den Schutzhosen für Mädchen und für Knaben. In Fig. 9 bis 16 sind einige der Schutzhosen dargestellt, welche unterschiedliche Schnitte aufweisen. Hier zeigen: Fig. 9 einen Damenslip, Fig. 10 einen Taillenslip für Damen, Fig. 11 ein Damenpanty, Fig. 12 einen Damenshort, Fig. 13 einen Herrenslip, Fig. 14 eine Classic für Herren, Fig. 15 einen Short und Fig. 16 einen Boxer. Die Damenhose gemäss Fig. 9 ist im Bereich der hochgeschnittenen Beinöffnungen 3 und 4 sowie im Bereich des Bundes 2 und des Mittelteiles 10 verziert, im dargestellten Fall mit Stickereien. Die Hose gemäss Fig. 10 weist Verzierungen auch im Bereich der Seitenteile 7 und 8 auf. Beim Classic-Short gemäss Fig. 14 ist ein Zierabschnitt im Mittelteil 10 eingesetzt.

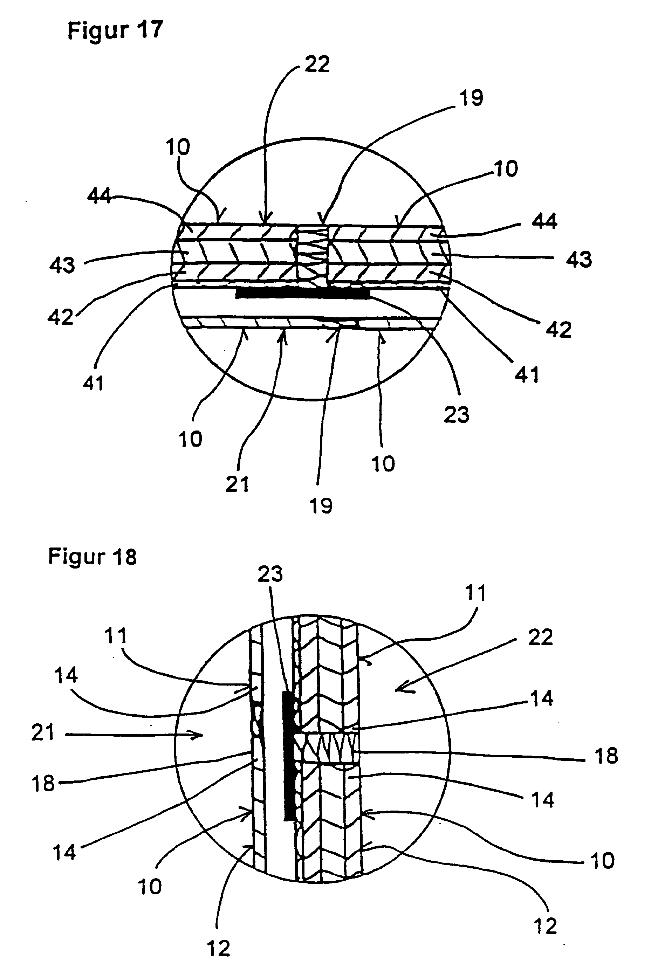

Um eine bessere Passform bei der Schutzhose zu erreichen, kann zumindest einer der Hosenteile 21 oder/und 22 im Schrittbereich mit einer Längsnaht 19 (Fig. 13 und 15) versehen sein. Fig. 17 zeigt in einem horizontalen Schnitt einen Ausschnitt aus dem Schrittbereichen der Mittelpartie 10 der Schutzhose. Die nebeneinander liegenden Abschnitte des Mittelteiles 10 der Innenhose 22, welche durch die Längsnaht 19 miteinander verbunden sind, weisen alle vier vorstehend genannten Schichten 41 bis 44 auf, weil die durch die Längsnaht 19 geschlossene Oeffnung im Mittelteil 10 durch blosses Entfernen eines Abschnittes des Mittelteiles 10 entstanden ist. Entsprechendes gilt für die Aussenhose 21. An der Aussenseite der Längsnaht 19 in der Innenhose 22 ist der bereits beschriebene Abdichtstreifen 23 angebracht.

Fig. 18 zeigt in einem vertikal verlaufenden Schnitt die Verbindungsstelle 14 innerhalb der aus zwei Abschnitten 11 und 12 bestehenden Mittelpartien 10 der Aussenhose 21 und der Innenhose 22, wenn es die Quernaht 14 bei der Innenhose 22 gibt. Die hier vorhandene Quernaht 20 ist an der Aussenseite der Innenhose 22 mit Hilfe eines weiteren Abdichtstreifens 23 abgedichtet.

## Patentansprüche

1. Schutzhose, die so ausgeführt ist, dass praktisch keine Flüssigkeit aus dieser austreten kann, mit einem Grundkörper (1), welcher eine Aussenhose (21) und eine Innenhose (22) umfasst, wobei die Innenhose (22) im Inneren der Aussenhose (21) angeordnet ist, **dadurch gekennzeichnet, dass** die jeweilige Teilhose (21;22) des Hosengrundkörpers (1) zwei einander gegenüberliegende Seitenteile (7,8) sowie einen sich da zwischen erstreckenden Mittelteil (10) umfasst, dass sich im unteren Abschnitt des jeweiligen Seitenteils (7,8) der Schutzhose ein Hosenbein (3,4) befindet, dass die Aussenhose (21) und die Innenhose (22) in den Bereichen der Taille und der Beinöffnungen (3,4) miteinander verbunden sind, dass das Material der Bestandteile (7,8,10) der Innenhose (22) als ein Sicherheitsstoff ausgeführt ist, dass dieser Sicherheitsstoff zwei Schichten (41,42) aus unterschiedlichen Materialien aufweist, dass sich an der Aussenseite des jeweiligen Bestandteiles (7,8,10) der Innenhose (22) eine Schicht (41) aus einem flüssigkeitsundurchlässigen Stoff befindet, dass sich diese Aussenschicht (41) an der der Aussenhose (21) zugewandten Seite der Innenhose (22) befindet, dass sich an der Innenseite der Innenhose (22) ein Innenstoff (42) befindet, welcher saugfähig und hautfreundlich ist, dass Abdichtbänder (23) vorgesehen sind, dass eine erste Gruppe dieser Abdichtbänder (23) jene Nahtstellen (14,15,16,20) überdeckt, an welchen zwei der Bestandteile (7,8,10,11,12) der Innenhose (22) aneinander treffen und dass diese Abdichtbänder (23) an der Aussenseite der Nahtstellen (14,15,16,20) der Innenhose (22) angeordnet sind.

2. Schutzhose nach Patentanspruch 1, **dadurch gekennzeichnet, dass** die flüssigkeitsundurchlässige Schicht (41) als eine Membrane aus Polyurethan ausgeführt ist und dass der saugfähige und hautfreundliche Stoff (42) aus Faden sein kann, welche ein Gemisch aus Baumwoll- und Polyesterfasern oder aus Baumwoll- und Polyamidfasern bilden.

3. Schutzhose nach Patentanspruch 1, **dadurch gekennzeichnet, dass** sich an der Innenseite des Innenstoffes (42) des Mittelteils (10) ein Stoff (43) befindet, welcher eine möglichst grosse Saugfähigkeit bzw. Kapazität zur Speicherung der austretenden Flüssigkeit aufweist, dass diese Stoffschicht (43) aus einem Doppelfrottee sein kann, dass diese Stoffschicht ausserdem entweder geruchshemmend ausgerüstet ist oder/und dass sie mit geruchshemmenden Garnen gewoben, gestrickt oder gewirkt sein kann, dass die dem Körper zugewandte Oberfläche der Saugstoffschicht (43) mit einer vierten Materialschicht (44) versehen ist, dass diese weitere Materialschicht (44) aus einem Stoff ist, welcher auch als Transportstoff bezeichnet werden kann, und dass dieser Transportstoff (44) aus geruchshemmenden Garnen hergestellt sein kann, wie z.B. aus Polyester oder aus Mikrofasern.

4. Schutzhose nach Patentanspruch 1, **dadurch gekennzeichnet, dass** die Abdichtbänder (23) Klebebänder sind oder dass sie als Bänder aus einem Material sind, welches unter der Einwirkung von Wärme weich bzw. halbflüssig wird, und dass die Abdichtstreifen (23) aus Polyurethan sein können.

5. Schutzhose nach Patentanspruch 1, **dadurch gekennzeichnet, dass** der Mittelteil (10) aus zwei länglichen und hintereinander geschalteten Abschnitten (11,12) zusammengesetzt ist, dass diese Abschnitte (11,12) unterschiedliche Längen aufweisen, dass der an der Vorderseite der Schutzhose liegende Mittelteilabschnitt (11) kürzer ist als der überwiegend im hinteren Bereich der Schutzhose liegende Mittelteilabschnitt (12), dass eine innere Verbindungsstelle (14) zwischen diesen Abschnitten (11,12) vorhanden ist, wo die Mittelteilabschnitte (11,12) miteinander verbunden sind, und dass diese innere Verbindungsstelle (14) im vorderen Bereich der Schutzhose liegt.

6. Schutzhose nach Patentanspruch 1, **dadurch gekennzeichnet, dass** die Umfangskontur des Mittelteiles (10) der Aussenhose (21) und der Innenhose (22) im wesentlichen die Form des Umrisses einer Sanduhr hat, dass die Aussenkontur des Mittelteiles (10) eine engste Stelle (25) aufweist, welche sich unmittelbar im Schrittbereich der Schutzhose befindet, dass an die jeweilige Endpartie dieser engsten Stelle (25) sich erweiterte Abschnitte (26,27) der genannten Kontur des Mittelteiles (10) anschliessen, dass am Ende des jeweiligen Breitabschnittes (26,27) sich jeweils eine der Quer- bzw. Endkanten des Mittelteiles (10) befindet und dass sich die Längskanten (15,16) des Mittelteiles (10) zwischen diesen Endkanten des Mittelteiles (10) erstrecken.

7. Schutzhose nach Patentanspruch 5, **dadurch gekennzeichnet, dass** die Innenseite, d.h. die dem Körper zugewandte Seite des Mittelteiles (10) der Innenhose (22) Mittel (30) zur Halterung von übereinander liegenden länglichen Einlagen (31.32) aufweist, dass der erste Teil dieser Haltemittel (30) als ein längliches Fach ausgeführt ist, wobei die Längsrichtung dieses Faches mit der Längsrichtung des Mittelteiles (10) zusammenfällt, dass dieses Fach durch eine Deckwand (33) aus einem textilen Stoff gebildet ist, welches auf der Ober- bzw. Innenseite der Mittelpartie (25) des Mittelteiles (10) aufliegt, und dass die Endpartien dieser Deckwand (33) im Bereich der breiten Partien (26,27) des Mittelteiles (10) liegen, wobei sie dabei die sich an die engste Stelle (25) anschliessenden Abschnitte der breiten Partien (26.27) des Mittelteiles (10) überdecken.

8. Schutzhose nach Patentanspruch 7, **dadurch gekennzeichnet, dass** Taschen (36,37) vorgesehen, welche für die Aufname der Endpartien der zw**ei**ten Einlage (32) bestimmt und ausgebildet sind, dass die jeweilige Tasche (36,37) durch einen im wesentlichen viereckförmigen Abschnitt aus einem textilen Stoff gebildet ist, welcher auf der dem Körper zugewandten Seite der Deckwand (33) des Faches befestigt ist.

9. Schutzhose nach Patentanspruch 8, **dadurch gekennzeichnet, dass** der erste Stoffabschnitt (36) auf dem vorderen Abschnitt der Deckwand (33) angebracht ist, wo sich die Einlegeöffnung (35) für die erste Einlage (31) befindet, wobei die vorne liegende Kante dieses ersten Stoffabschnittes (36) mit der frei stehenden Vorderkante (35) der Deckwand (33) praktisch bündig ist, dass die Vorderkante und die Seitenkanten des ersten Stoffabschnittes (36) mit dem Stoff der Deckwand (33) verbunden sind, beispielsweise durch Nähte, dass diese Tasche (36) eine Öffnung aufweist, durch welche eine der Endpartien der zweiten Einlage (32) in diese Tasche (36) eingeführt werden kann, dass der zweite Stoffabschnitt (37) auf dem hinten liegenden Abschnitt der Deckwand (33) angebracht ist, wo sich die an das Material der Innenhose (22) angeschlossene hintere Querkante (34) des Faches (33) befindet, dass die hinten liegende Kante dieses Stoffabschnittes (37) zusammen mit der hier liegenden Hinterkante (34) der Deckwand (33) an das Material der Innenhose (22) angeschlossen ist, und dass eine Öffnung dieser Tasche (37) im Bereich der genannten Vorderkante (39) vorhanden ist, durch welche die zweite Endpartie der zweiten Einlage (32) in diese Tasche (37) eingeführt werden kann.

10. Schutzhose nach Patentanspruch 1, **dadurch gekennzeichnet, dass** die Bestandteile (7,8,10) der Aussenhose (21) durch einen Oberstoff gebildet sind, welcher bei der Herstellung von Unterhosen normalerweise verwendet wird, und dass dieser Stoff ein Baumwollstoff sein kann.

11. Schutzhose nach Patentanspruch 3, **dadurch gekennzeichnet, dass** zumindest eine der Teilhosen (21 oder/und 22) im Schrittbereich mit einer Längsnaht (19) versehen ist, dass die nebeneinander liegenden Abschnitte des Mittelteils (10) der Innenhose (22), welche durch die Längsnaht (19) miteinander verbunden sind, die mehrschichtige Struktur (41 bis 44) aufweisen und dass dies auch für die Aussenhose (21) zutreffen kann.

12. Schutzhose nach Patentanspruch 1, **dadurch gekennzeichnet, dass** die Seitenteile (7.8) der Innenhose (22) die ersten zwei Schichten (41,42) aufweisen, dass der Mittelteil (10) der Innenhose (22) die vier Schichten (41 bis 44) aufweist, dass die Verbindungsstellen der Teile (7,8,10) der Innenhose (22) in einem Bereich liegen, wo sich auch die Verbindungsstellen (16) der Teile (7,8,10) der Aussenhose (21) befinden, dass Verbindung der Teile (7,8,10) der Innenhose (22) mit Hilfe einer Naht (20) erfolgt, auf welcher der Abdichtstreifen (23) angebracht ist und dass dieser Streifen (23) breiter ist als die Naht (20).

13. Verfahren zur Herstellung der Schutzhose gemäss Patenanspruch 1, **dadurch gekennzeichnet, dass** die Aussenhose (21) und die Innenhose (22) des Grundkörpers (1) der Schutzhose voneinander getrennt hergestellt werden, dass dann die Innenhose (22) in die Aussenhose (21) gelegt wird und dass diese Teilhosen (21,22) in den Bereichen des Hosenbundes (2) und der Hosenbeinöffnungen (3,4) zusammen verbunden werden.

## Claims

1. Protective pants configured such that virtually no fluid can escape from them, with a basic body (1) having an outer pants (21) and an inner pants (22), the inner pant arranged in an interior of the outer pants (21), **characterized in that** the said pants parts (21;22) of the basic body (1) of the pants have two mutually opposite side parts (7, 8) and a central part (10) arranged therebetween, that they are located in the lower portion of the said side parts (7, 8) of the protective pants of a pant leg (3, 4), that the outer pants (21) and the inner pants (22) are bound together in the region of the waistline and the leg opening (3, 4), that the material of the components (7, 8, 10) of the inner pants (22) is made of a security fabric, that the security fabric is made up of two layers (41, 42) of different materials, that on the outside of the said components (7, 8, 10) of the inner pants (22) it is made up of a layer (41) of a fluid impermeable material, that the outer layer (41) is disposed on side of the inner pants (22) facing the outer pants (21), that an inner material (42) is disposed on the inner side of the inner pants (22), which is absorbent and comfortable to the skin, that sealing strips (23) are provided, **in that** a first group of these strips (23) covers over those seams (14, 15, 16, 20) at which two constituent parts (7, 8, 10, 11, 12) of the inner pants (22) meet, and **in that** said strips (23) are arranged on the outer side of the seams (14, 15, 16, 20) of the inner pants (22).

2. Protective pants according to claim 1, **characterized in that** the fluid impermeable layer (41) is a membrane made of polyurethane and that absorbent and skin-compatible material (42) may be made of filaments, which may contain a mixture of cotton and polyester fibers or of cotton and polyamid fibers.

3. Protective pants according to claim 1, **characterized in that,** located on the inner side of the inner material (42) of the central part (10) is a material (43) which has the highest possible absorbency and/or capacity to store the outflowing fluid, that the material layer (43) may be made of a double terry material, that the material layer (43) may either be provided with odor-inhibiting means and/or it may be woven or knitted with odor-inhibiting yarns, that the surface of the absorbent-material layer (43) which is directed toward the body is provided with a fourth material layer (44) made of a material which may also be referred to as a transporting material, this material (44) may likewise be produced from odor-inhibiting yarns, for example of polyester or of microfibers

4. Protective pants according to claim 1, **characterized in that** the sealing strips (23) may be configured as adhesive strips or as strips of a material which becomes soft or semi-liquid under the action of heat, and that the sealing strips (23) can be made of Polyurethan.

5. Protective pants according to claim 1, **characterized in that** the central part (10) is made up of two elongate sections (11, 12) arranged one behind the other, **in that** said sections (11, 12) are of different lengths, **in that** the central-part section (11), which is located on the front side of the protective pants, is shorter than the central-part section (12), which is located predominantly in the rear region of the protective pants, **in that** an inner connecting location (14) is provided between the sections (11, 12), where the central-part sections (11, 12) are connected to one another, and **in that** said inner connecting location (14) is located in the front region of the protective pants.

6. Protective pants according to claim 1, **characterized in that** the peripheral contour of the central part (10) of the outer pants (21) and of the inner pants (22) is essentially in the form of the outline of an hourglass, **in that** the outer contour of the central part (10) has a narrowest location (25), which is located directly in the crotch region of the protective pants, **in that** the respective end portion of said narrowest location (25) is adjoined by widened sections (26, 27) of said contour of the central part (10), **in that** in each case one of the transverse or end edges of the central part (10) is located at the end of the respective wide section (26, 27), and **in that** the longitudinal edges (15, 16) extend between said end edges of the central part (10).

7. Protective pants according to claim 5, **characterized in that** that side of the central part (10) of the inner pants (22) which is directed toward the body has means (30) for securinq elongate liners (31, 32) located one above the other, **in that** the first part of the retaining means (30), which is designed for accommodating the first liner (31), is configured as an elongate compartment, the longitudinal direction of said compartment coinciding with the longitudinal direction of the central part (10), **in that** said compartment is formed by an upper wall (33) which rests on the top side or inner side, inter alia, of the central potion (25) of the central part (10), and **in that** the end portions of said upper wall (33) are located in the region of the wide portions (26, 27) of the central part (10), wherein the widened sections (26, 27) adjoined to the narrowest location (25) of the central part (10) are covered.

8. Protective pants according to claim 7, **characterized in that** pockets (36, 37) are provided which are intended and designed for accommodating the end portions of the second liner (32), **in that** said pockets (36, 37) are provided on the upper wall (33) of the compartment, and **in that** in each case one of these quadrangular formed textile pockets (36, 37) is directed to the body and located in one of the end regions of the upper wall (33) of the compartment, where the respective pocket is fastened.

9. Protective pants according to claim 8, **characterised in that** the first material section (36) is provided on the front section of the upper wall (33), where the insertion opening (35) for the first liner (31) is located, by which the front edge of said first material section (36) is virtually flush with the free front edge (35) of the upper wall (33), that the front edge and the side edges of said first material section (36) are connected, for example by sewing, to the material of the upper wall (33), that the pocket (36) has an opening through which one of the end portions of the second liner (32) can be introduced into said pocket (36), that the second material section (37) is provided on the rear section of the upper wall (33), where the rear transverse edge (34) of the compartment (33) is located, said transverse edge adjoining the material of the inner pants (22), that the rear edge of said material section (37) is connected to the material of the inner pants (22) together with the rear edge (34), located here, of the upper wall (33), and that the opening for this pocket (37) is provided in the region of said front edge (39), and the second end portion of the second liner (32) can be introduced into said pocket (37) through said opening.

10. Protective pants according to claim 1, **characterised in that** the components (7, 8, 10) of the outer pants (21) are made of a outer fabric which is normally used in the manufacturing of underpants, and that this material may be cotton.

11. Protective pants according to claim 3, **characterised in that** at least one of the pants (21 and/or 22) in the crotch region is furnished with a longitudinal seam (19), so that the adjacent sections of the central part (10) of the inner pants (22) which are connected to one another by the longitudinal seam (19) have a multi-layered structure (41-44) and that this can be applied to the outer pants (21).

12. Protective pants according to claim 1, **characterised in that** side parts (7, 8) of the inner pants (22) is made up of the first two layers (41, 42), that the central part (10) of the inner pants (22) has all four layers (41 - 44), that the connection of the parts (7, 8, 10) of the inner pants (22) is disposed in the region of the connecting location (16) on the outer pants (21), the connection of the parts (7, 8, 10) of the inner pants (22) with the aid of a seam (20), on which the sealing strip (23) is disposed, and that sealing strip (23) is wider than the seam (20).

13. A method of producing the protective pants as claimed in patent claim 1, **characterized in that** the outer pants (21) and the inner pants (22) of the basic body (1) of the protective pants are produced separately from one another, **in that** the inner pants (22) is then positioned in the outer pants (21), and **in that** said pants (21, 22) are connected together in the regions of the pants waistband (2) and of the pants-leg openings (3, 4).

## Revendications

1. Culotte de protection configurée de manière à ce que sensiblement aucun fluide puisse s'échapper de cette dernière, avec un corps de base (1) comportant une culotte extérieure (21) et une culotte intérieure (22), ladite culotte intérieure (22) étant arrangée à l'intérieur de ladite culotte extérieure (21), **caractérisée en ce que** chacune des culottes partielles (21 ;22) dudit corps de base de culotte (1) comprend deux parties latérales (7,8) opposées l'une à l'autre ainsi qu'une partie centrale (10) s'étendant entre ces dernières, **en ce qu'**une jambe de culotte (3,4) est arrangée dans la section inférieure de chacune desdites parties latérales (7,8) de ladite culotte de protection, **en ce que** ladite culotte extérieure (21) et ladite culotte intérieure (22) sont reliées l'une à l'autre dans les régions de la taille et des ouvertures desdites jambes (3,4), **en ce que** le matériau des parties (7,8,10) de ladite culotte intérieure (22) est réalisé sous forme de tissu de sécurité, **en ce que** ledit tissu de sécurité comprend deux couches (41,42) en matériaux différents, **en ce qu'**une couche (41) en matériau imperméable aux fluides est arrangée du côté extérieur desdites parties (7,8,10) de ladite culotte (22), **en ce que** ladite couche extérieure (41) est arrangée du côté de ladite culotte intérieure (22) et en regard de ladite culotte extérieure, **en ce qu'**un matériau intérieur (42) qui est absorbant et agréable pour la peau est arrangé du côté intérieur de ladite culotte intérieure (22), **en ce que** des rubans d'étanchéité (23) sont prévus, **en ce qu'**un premier groupe desdits rubans d'étanchéité (23) couvre des coutures (14,15,16,20) auxquelles deux parties (7,8,9,10,11,12) de ladite culotte intérieure (22) se joignent, et **en ce que** lesdits rubans d'étanchéité (23) sont arrangés du côté extérieur desdites coutures (14,15,16,20) de ladite culotte intérieure (22).

2. Culotte de protection selon la revendication 1, **caractérisée en ce que** ladite couche imperméable aux fluides (41) est réalisée en tant que membrane en polyuréthane, et **en ce que** ledit matériau (42) absorbant et agréable pour la peau peut être composé de fils qui peuvent être une mixture de fibres de coton et de polyester ou de fibres de coton et de polyamide.

3. Culotte de protection selon la revendication 1, **caractérisée en ce que** du côté intérieur dudit matériau intérieur (42) de ladite partie centrale (10), un matériau (43) se trouve dont l'absorptivité (capacité à absorber de liquides) est la plus grande possible pour stocker du liquide qui s'écoule, **en ce que** ladite couche de matériau (43) peut être un tissu éponge double, **en ce que** ladite couche de matériau peut en outre soit être équipée de manière à inhiber les odeurs et/ou être tissée ou tricotée, **en ce que** la surface orientée vers le corps, de la couche de matériau absorbant (43) est pourvue d'une quatrième couche de matériau (44) réalisée en un matériau pouvant être appelé matériau transporteur (44), et **en ce que** ledit matériau transporteur (44) peut être fabriqué par des fils textiles inhibant les odeurs, notamment en polyester ou en microfibre.

4. Culotte de protection selon la revendication 1, **caractérisée en ce que** lesdits rubans d'étanchéité (23) sont des rubans adhésifs, ou **en ce qu'**ils sont fabriqués en matériau qui s'amollit ou qui devient semi-liquide sous l'influence de la chaleur, et **en ce que** lesdits rubans d'étanchéité (23) peuvent être en polyuréthane.

5. Culotte de protection selon la revendication 1, **caractérisée en ce que** ladite partie centrale (10) est composée de deux sections oblongues (11,12) et arrangées en série, **en ce que** lesdites sections (11,12) présentent des longueurs différentes, **en ce que** la section partielle centrale (11) disposée du côté avant de la culotte de protection est plus courte que la section partielle centrale (12) disposée en général du côté arrière de la culotte de protection, **en ce qu'**il y a un point de jonction interne (14) entre lesdites sections (11,12) où lesdites sections partielles centrales (11,12) sont raccordées les unes aux autres, et **en ce que** ledit point de jonction interne (14) est disposé dans la région avant de la culotte de protection.

6. Culotte de protection selon la revendication 1, **caractérisée en ce que** le contour périphérique de ladite partie centrale (10) de ladite culotte extérieure (21) et de ladite culotte intérieure (22) présente sensiblement la forme de la silhouette d'un sablier, **en ce que** le contour extérieur de ladite partie centrale (10) comprend un point le plus étroit (25) qui se trouve directement dans la région de l'entrejambe de la culotte de protection, **en ce qu'**à la partie d'extrémité dudit point le plus étroit (25) sont raccordées des sections élargies (26,27) dudit contour de ladite partie centrale (10), **en ce qu'**à l'extrémité de ladite section large (26,27) se trouve respectivement l'une des bordures transversales ou d'extrémité de ladite partie centrale (10), et **en ce que** lesdites bordures longitudinales (15,16) de ladite partie centrale (10) s'étendent entre lesdites bordures d'extrémité de ladite partie centrale (10).

7. Culotte de protection selon la revendication 5, **caractérisée en ce que** le côté intérieur, c'est-à-dire le côté tourné vers le corps, de ladite partie centrale (10) de ladite culotte intérieure (22) comprend des moyens (30) pour fixer des inserts oblongs (31,32) disposés les uns sur les autres, **en ce que** la première partie desdits moyens de fixation (30) est réalisée sous forme d'un compartiment oblong dont la direction longitudinale coïncide avec la direction longitudinale de ladite partie centrale (10), **en ce que** ledit compartiment est formé par une paroi supérieure (33) en matériau textile qui repose sur le côté supérieur ou le côté intérieur de la région centrale (25) de ladite partie centrale (10), et **en ce que** lesdites parties d'extrémité de ladite paroi supérieure (33) sont positionnées dans la région desdites parties larges (26,27) de ladite partie centrale (10), en couvrant les sections, raccordées au point le plus étroit (25), desdites parties larges (26,27) de ladite partie centrale (10).

8. Culotte de protection selon la revendication 7, **caractérisée en ce que** des poches (36,37) destinées à et formées pour recevoir les parties d'extrémité du second insert (32) sont prévues, **en ce que** chacune desdites poches (36,37) est formée par une section sensiblement quadrangulaire en matériau textile et attachée du côté orienté vers le corps, de ladite paroi supérieure (33) dudit compartiment.

9. Culotte de protection selon la revendication 8, **caractérisée en ce que** la première section (36) est attachée sur la section avant de ladite paroi supérieure (33) où se trouve l'ouverture d'insertion (35) pour le premier insert (31), de sorte que la bordure avant de ladite première section (36) et la bordure avant libre (35) de ladite paroi supérieure (33) sont pratiquement au même niveau, **en ce que** ladite bordure avant et les bordures latérales de ladite première section (36) sont reliées, par exemple par des coutures, au matériau de ladite paroi supérieure (33), **en ce que** ladite poche (36) comprend une ouverture par laquelle les parties d'extrémité dudit second insert (32) peuvent être introduits dans ladite poche (36), **en ce que** la seconde section (37) est attachée sur la section arrière de ladite paroi supérieure (33) où se trouve la bordure transversale arrière (34), raccordée au matériau de ladite culotte intérieure, dudit compartiment (33), **en ce que** la bordure arrière de ladite section (37) est raccordée au matériau de ladite culotte intérieure (22) conjointement avec la bordure arrière (34), positionnée à cet endroit, de ladite paroi supérieure (33), et **en ce qu'**une ouverture de ladite poche (37) est prévue dans la région de ladite bordure avant (39) par laquelle ladite seconde partie d'extrémité dudit second insert (32) peut être introduite dans ladite poche (32).

10. Culotte de protection selon la revendication 1, **caractérisée en ce que** lesdites parties (7,8,10) de ladite culotte extérieure (21) sont réalisées par un textile extérieur que l'on utilise normalement lors de la fabrication de caleçons, et **en ce que** ledit textile peut être du coton.

11. Culotte de protection selon la revendication 3, **caractérisée en ce qu'**au moins une desdites culottes partielles (21 et/ou 22) est pourvue d'une couture longitudinale (19) dans la région de l'entrejambe, **en ce que** lesdites sections adjacentes de la partie centrale (10) de ladite culotte intérieure (22), qui sont reliées l'une à l'autre par ladite couture longitudinale (19), comprennent la structure multicouche (41 à 44), ce qui peut aussi être le cas pour ladite culotte extérieure (21).

12. Culotte de protection selon la revendication 1, **caractérisée en ce que** les parties latérales (7,8) de ladite culotte intérieure (22) comprennent les premières deux couches (41,42), **en ce que** la partie centrale (10) de ladite culotte intérieure (22) comprend les quatre couches (41 à 44), **en ce que** les positions de raccordement desdites parties (7,8,10) de ladite culotte intérieure (22) se situent dans une région où se trouvent aussi lesdites positions de raccordement (16) desdites parties (7,8,10) de ladite culotte extérieure (21), **en ce que** le raccordement desdites parties (7,8,10) de ladite culotte intérieure (22) est effectué par une couture (20) sur laquelle est positionné ledit ruban d'étanchéité, et **en ce que** ledit ruban (23) est plus large que ladite couture (20).

13. Procédé de fabrication de la culotte de protection revendiquée dans la revendication 1, **caractérisé en ce que** ladite culotte extérieure (21) et ladite culotte intérieure (22) dudit corps de base (1) de ladite culotte de protection sont fabriquées séparément; **en ce qu'**ensuite, ladite culotte intérieure (22) est positionnée dans ladite culotte extérieure (21); et **en ce que** lesdites culottes partielles (21,22) sont reliées l'une à l'autre dans les régions de la ceinture de la culotte (2) et des ouvertures des jambes de la culotte (3,4).
